# EUROPEAN PATENT APPLICATION

(11) **EP 3 831 417 A1**
(43) Date of publication of application: **09.06.2021**
(21) Application number: 21460005.8
(22) Date of filing: 15.01.2021
(51) Int. Cl.: A61L 27/06, A61L 27/34, A61L 27/54

(54) **METHOD FOR PRODUCING POLYMER LAYERS ON THE SURFACE OF TITANIUM OR TITANIUM ALLOYS BASED ON A POLYMER WITH PHOSPHATES**

(30) Priority: 21.01.2020 PL 43265220
(71) Applicant: Politechnika Slaska, 44-100 Gliwice (PL)
(72) Inventor: Kazek-Kesik, Alicja, 41-310 Dabrowa Górnicza (PL); Smiga-Matuszowicz, Monika, 44-121 Gliwice (PL); Wojciech, Simka, 40-748 Katowice (PL)

(57) **Abstract**

The method of formation polymer layers on the surface of titanium or titanium alloys based on polymer with phosphates, characterized in that the titanium or titanium alloy is anodically oxidized in a water bath containing calcium hypophosphite with a concentration of 0.001 mol/dm3-10 mol/dm3 using a voltage ranging from 0.1V-800 V, and an anodic current density in the range of 5-500 A / dm2 and the anodized titanium or titanium alloy is then placed in a polymer solution based on an adipic acid derivative and a solvent, preferably dichloromethane, and insoluble zinc phosphate compounds preferably (Zn3(PO4)2) and / or copper phosphates preferably (Cu3(PO4)2)) and / or silver phosphates preferably (Ag3PO4), and dry.

## Description

The subject of the invention is the method of formation polymer layers on the surface of titanium or titanium alloys based on polymer with phosphates, especially for use as coatings in implantable materials.

Among biodegradable polymers, aliphatic polyanhydrides are the group with the highest susceptibility to hydrolysis, which precludes them from many traditional technical applications. Hydrolytic instability of polyanhydrides is the feature beneficial in biomedical applications, especially in drug-controlled release systems, where polyanhydrides act as a matrix/carrier of the drug. Research on such systems was started by R. Langer et al. in the 1980s [Rosen HB, Chang J, Wnek GE, Linhardt RJ, Langer R. Bioerodible polyanhydrides for controlled drug delivery. Biomaterials 1983, 4, 131-134]. Many years of research work have led to the elaboration of two systems currently approved for use in clinical practice. These systems include Gliadel® - a system of local delivery of chemotherapeutic agents (BCNU or carmustine) in the therapy of brain cancer and Septacin™ - an implant for local delivery of gentamicin in bacterial infections of bone tissue [Jain JP, Kumar N, Chitkara D. Polyanhydrides as localized drug delivery carrier: An update. Expert Opin. Drug Deliv. 2008, 5, 889-907].

Poly(adipic anhydride) (PADA), first synthesized more than 90 years ago [Hill JW. J. Am. Chem. Soc. 1930, 52, 4110], is one of many aliphatic polyanhydrides tested in the 1990s for use as a matrix in controlled drug release [Albertsson A-C, Calfors J, Sturesson C. Preparation and characterization of poly(adipic anhydride) microspheres for ocular drug delivery. J. Appl. Polym. Sci. 1996, 62, 695-705; Edmund U, Albertsson A-C, Singh SK, Fogelberg I, Lundgren BO. Sterilization, storage stability and in vivo biocompatibility of poly(trimethylene carbonate)/poly(adipic anhydride) blends. Biomaterials 2000, 21,945-955].

A method of poly(adipic anhydride) synthesis, which creates the polymer that can potentially be used as a drug-releasing matrix, is a known from the publication "In vitro release of clomipramine HCl and buprenorphine HCl from poly adipic anhydride (PAA) and poly trimethylene carbonate (PTMC) blends". (Dinarvand R., Alimorad M.M., Amanlou M., Akbari H.; J Biomed Mater Res A. 2005 Oct. 1;75(1):185-91). Another way of synthesizing poly(adipic anhydride) is presented in the publication "Synthesis of Poly(Adipic Anhydride) by Use of Ketene" (Ann-Christine Albertsson & Stefan Lundmark, Journal of Macromolecular Science: Part A - Chemistry 25, 1986, 247-258). The published data show that hydrolytic degradation of PADA is a surface process which proceeds with a high rate, resulting in the formation of adipic acid - a product of hydrolysis of anhydride groups in the polymer. Under *in vivo* conditions, adipic acid is metabolized in the process of β-oxidation of fatty acids.

From GB1436473, one can see that an application of adipic acid is in the production of cement-containing ceramic materials for construction applications. However, there are no reports in the literature regarding the application of poly(adipic anhydride) for the formation of oxide-polymer coatings on bone implant materials. EP3488877 shows the formation of porous polymer coatings on the implant surface, which consist of a polymer layer and a layer containing a biocompatible material. This document reports a three-layered material which may contain collagen in its composition. The application of polymers and cyclic peptide ligands to coat implantable materials such as stents used in cardiac surgery has also been seen (US2019247539). Polymeric materials are also utilized in the preparation of bone scaffolds (scaffolds) for the regeneration and filling of bone defects (US2019255221). From the document WO2018076003, a method for obtaining composites based on magnesium and polymers is known, where the polymer is a part of the obtained composite but is not formed to its surface.

In patent application P.425256, a method of formation polymer oxide coatings is demonstrated, where a polymer based on sebacic acid or other carboxylic acids was used. This method differs from the proposed method for the preparation of coatings using an adipic acid-based polymer in that adipic acid is a different chemical compound. In contrast, polymer degradation time in physiological fluids is much shorter, other degradation products are obtained, and biologically-active substances and other active substances are released in a much shorter time. On the basis of the conducted research, it was found that depending on the substance used, the biologically-active degradation time is the degradation time of the polymer layer composed of poly(adipic anhydride).

The aim of the invention is to develop such a method of formation polymer layers on titanium or titanium alloy surfaces, which will create a material from which a biologically-active or bioactive substance is released from the upper layer in a short amount of time.

The essence of the invention is the method of formation polymer layers on the surface of titanium or titanium alloys based on polymer with phosphates characterized in that the titanium or titanium alloy is anodized in bath composed of calcium hypophosphite in concentrations between 0.001 mol/dm³-10 mol/dm³ using an applied voltage between 0.1V-800 V and a current density between 5-500 A/dm². The anodized titanium or titanium alloy is immersed in a polymer solution based on the adipic acid derivative and a solvent, preferably dichloromethane and insoluble zinc phosphate compounds, preferably (Zn3(PO4)2) and/or copper phosphates, preferably (C_{U3}(PO₄)₂)) and/or silver phosphates, preferably (Ag₃PO₄), and dry. Optimal anodization times are between 1 s and 10 minutes. The polymer is solution is an adipic acid-based polymer with a poly(adipic anhydride) derivative and a solvent in a ratio of 1:100-20:100. To coat the material with the polymer, the titanium or titanium alloy is placed in the polymer solution, which is contained in a closed container. The materials are then shaken to complete the coating process. Preferably, the insoluble zinc phosphate compounds, preferably (Zn₃(PO₄)₂) and/or copper phosphates, preferably (Cu₃(PO₄)₂)) and/or silver phosphates, preferably (Ag₃PO₄) in concentration form 0.1 g/L to 100 g/L as a solution or powder.

The invention shows that the degradation time of the polymer layer obtained on the surface of previously-anodized titanium implants ranges from the first few minutes to 48 hours. After the first 8 hours of the implant's immersion with a polymer-oxide coating based on adipic acid, the amount of adipic acid formed as a polymer degradation product is over 60%. The proposed method of formation oxide-polymer layers based on adipic acid derivatives makes it possible to obtain a material from which the upper release layer is a biologically active substance or a bioactive chemical substance, which can be released in the first 30 minutes.

The invention is shown in the following examples, which do not exhaust all the embodiments according to the invention.
**Example I:** In the titanium (Grade 4) surface treatment, an anodized bath is composed of 0.5 mol/dm³ calcium hypophosphite. The process is carried out at 5°C, using a current density of 100 mA/cm², a voltage of 300 V, and a process time of 5 min. Then, on the anodized metal surface, a polymer layer is formed from 1 % (w/v) poly(adipic anhydrite) PADA solution in dichloromethane in an amount of 1 g of polymer in 100 ml of dichloromethane. To the polymer solution a insoluble zinc(II) phosphate (Zn₃(PO₄)₂) in concentration 2 g/L was added. The solution was mixed for 15 s.
   The immersion rate of the oxidized sample into the polymer solution is 2 cm/min, the residence time of the oxidized sample in the polymer solution is 30 seconds, the drawing speed of the sample is 2 cm/min, and the drying time at ambient temperature is 30 minutes.
**Example II**: In the titanium alloy Ti-6A1-4V surface treatment, an anodizing bath is composed of 4 mol/dm³ calcium hypophosphite. The process is carried out at 15°C using current density of 100 mA/cm², a voltage of 600 V and a process time of 5 minutes. Then, on the anodized metal surface, a polymer layer is formed from 1.5% (w/v) poly(adipic anhydrite) PADA solution in dichloromethane in an amount of 0.2 g of polymer in 10 ml of dichloromethane and insoluble copper(II) phosphate (Cu₃(PO₄)₂) in concentration 0.5 g/ dm³. The solution was mixed for 20 s.
   The immersion rate of the oxidized sample into the polymer solution is 1 cm/min, the residence time of the oxidized sample in the polymer solution is 60 seconds, the drawing speed of the sample is 1 cm/min, and the drying time at ambient temperature is 5 minutes.
**Example III**: In the titanium alloy Ti-13Nb-13Zr surface treatment, an anodizing bath is composed of 10 mol/dm³ calcium hypophosphite. The process is carried out at 35°C using a current density of 150 mA/cm², a voltage of 450 V, and process time of 5 minutes. The anodized titanium alloy is then placed in a container containing a 10% (co)polyanhydride polymer solution in an amount of 0.2 g in 10 ml of dichloromethane. and insoluble silver(I) phosphate (Ag3(PO₄)₂) in concentration 25 g/L.
   The container is closed, and the operator shakes the closed container manually for 10 seconds.
**Example IV:** Example IV differs from Example I, that instead of zinc(II) phosphate(V), a mixture of an insoluble compound of zinc(II) phosphate (Zn3(PO₄)₂) and copper(II) phosphate(V) is added (Cu₃(PO₄)₂)) in concentration of 5 g/L and 10 g/L, respectively. The solution was stirred for 60 seconds.
Example V: Example V differs from Example II, that instead of copper phosphate, a mixture of an insoluble silver(I) phosphate (Ag3(PO₄)₂) and copper(II) phosphate(V) compound is added (CU₃(PO₄)₂)) in amounts of 2 g/L and 5 g/L, respectively, and mixed for 60 seconds.
Example VI. Example VI differs from Example III in that a silver phosphate is replaced by a mixture of zinc(II) phosphate (Zn₃(PO₄)₂), silver(I) phosphate (Ag₃(PO₄)₂) and copper (II) phosphate (Cu₃(PO₄)₂) in a concentration of 2g/L and 5 g/L and 7 g/L, respectively.

## Claims

1. The method of formation polymer layers on the surface of titanium or titanium alloys based on polymer with phosphates, **characterized in that** the titanium or titanium alloy is anodically oxidized in a water bath containing calcium hypophosphite with a concentration of 0.001 mol/dm³-10 mol/dm³ using a voltage ranging from 0.1V-800 V, and an anodic current density in the range of 5-500 A / dm² and the anodized titanium or titanium alloy is then placed in a polymer solution based on an adipic acid derivative and a solvent, preferably dichloromethane, and insoluble zinc phosphate compounds preferably (Zn₃(PO₄)2) and / or copper phosphates preferably (Cu₃(PO₄)₂)) and / or silver phosphates preferably (Ag₃PO₄), and dry.

2. The method of claim 1, wherein the anodization process carried out in time between 1 second and 10 minutes.

3. The method of claim 1 or 2, wherein the polymer solution is based on adipic acid and solvent in proportion 1:100-20:100.

4. The method of claim 1, 2 or 3 wherein the adipic acid derivative is poly(adipic anhydride).

5. The method of claim 1, 2 or 3 wherein the adipic acid derivative is (*co*)polyanhydride.

6. The method of claims 1-5, wherein the titanium or titanium alloy is placed into a solution via a dip coating technique.

7. The method of claims 1-5, wherein the titanium or titanium alloys is placed into a closed chamber and shaken.

8. The method of claims 1-7, wherein to the polymer solution the insoluble zinc phosphate compounds, preferably (Zn₃(PO₄)₂) and/or copper phosphates, preferably (Cu₃(PO₄)₂)) and/or silver phosphates preferably (Ag₃PO₄) as solution or powder in concentration from 0.1 g/L to 100 g/L,
